# EUROPEAN PATENT APPLICATION

(11) **EP 4 813 290 A1**
(43) Date of publication of application: **30.09.2026**
(21) Application number: 24903851.4
(22) Date of filing: 14.06.2024
(51) Int. Cl.: A61B 5/11, G06N 3/0464

(54) **METHOD AND SYSTEM FOR AUTOMATICALLY EVALUATING MOVEMENT SMOOTHNESS OF INFANT**

(30) Priority: 12.12.2023 KR 20230180033
(71) Applicant: Chung Ang University Industry Academic Cooperation Foundation, Seoul 06974 (KR)
(72) Inventor: LEE, Woo Hyung, Seoul 06511 (KR); LEE, Yae Lim, Seoul 04728 (KR); SHIN, Hyun Lee, Seoul 06001 (KR)
(74) Representative: BCKIP Part mbB
(86) International application number: PCT/KR2024/008221
(87) International publication number: WO 2025/127279

(57) **Abstract**

A method for automatically evaluating the smoothness of voluntary movements of an infant, according to one embodiment of the present invention, comprises the steps of: obtaining a two-dimensional video of the infant who is moving voluntarily; obtaining position coordinate data of joints of the infant for each frame of the video by using a deep learning-based pose prediction model; and quantifying the smoothness of the voluntary movements of the infant on the basis of the position coordinate data of the joints.

## Description

### THECHNICAL FIELD

The present invention relates to a method and system for automatically evaluating movement smoothness of infant.

### BACKGROUND OF THE INVENTION

Cerebral palsy is a general term for motor dysfunction caused by brain lesions that occur before, during, or after birth, when the brain is still immature. Its main symptoms include muscle control and motor function abnormalities, as well as delayed motor development. To minimize potential future disabilities, it is important to predict cerebral palsy or delayed motor development early and implement appropriate therapeutic interventions at an early stage.

Traditionally, the General Movement Assessment (GMA), developed by Pretzel, has been recommended as a method for predicting the risk of cerebral palsy or motor development delay in infants, particularly preterm infants. However, the conventional General Movement Assessment relies on video recordings of the voluntary movements of infants, which are then evaluated and scored by a trained assessor who has obtained the relevant certification. Consequently, the process can be time-consuming depending on the assessor's proficiency, is highly dependent on the assessor's expertise, and presents challenges in making quantitative judgments.

Although engineers have long attempted to perform quantitative assessments of voluntary movement, no evaluation method has been developed that is successful enough for clinical use, and such efforts have remained at the research level.

Meanwhile, the development of new parameters showing a significant association with the subsequent occurrence of motor development delay in infants is crucial as a preliminary step toward the future development of automated motor development delay prediction programs. By discovering new parameters associated with the risk of cerebral palsy or motor development delay and quantifying them using machine learning-based automated methods, it can be provided additional information that may influence clinical judgment.

In other words, there is a need for effective automated movement assessment methods and systems capable of early prediction of cerebral palsy or motor development delay in infants by quantifying new parameters associated with these conditions using machine learning-based quantitative methods.

### DETAILED DESCRIPTION OF THE INVENTION

### TECHNICAL PROBLEM

The technical objective of the present invention is to solve the problems associated with the prior art described above. The technical objective sought to be achieved by this embodiment is not limited to the technical objective described above; other technical objectives may be inferred from the embodiments described below.

### TECHNICAL SOLUTION

As a technical means for achieving the aforementioned technical objectives, a method for automatically evaluating the smoothness of voluntary movements of an infant may comprise the steps of: obtaining a two-dimensional video of the infant who is moving voluntarily; obtaining position coordinate data of joints of the infant for each frame of the video by using a deep learning-based pose prediction model; and quantifying the smoothness of the voluntary movements of the infant on the basis of the position coordinate data of the joints.

Wherein the method may further comprise the steps of: obtaining angle data and angular velocity data of the joints based on the position coordinate data of the joints, after obtaining the position coordinate data of the joints,

Wherein the quantifying the smoothness of the voluntary movements of the infant comprises, quantifying the smoothness of the voluntary movements of the infant based on the angle data and the angular velocity data of the joints.

Wherein the quantifying the smoothness of the voluntary movements of the infant may comprise, measuring a log dimensionless jerk (LDJ) and spectral arc length (SPARC) based on the angle data and the angular velocity data of the joints.

Wherein the position coordinate data for the joints may comprise, the position coordinate data for at least one of the left shoulder joint, the right shoulder joint, the left elbow joint, the right elbow joint, the left wrist joint, the right wrist joint, the left hip joint, the right hip joint, the left knee joint, the right knee joint, the left ankle joint, and the right ankle joint.

A system for automatically evaluating smoothness of voluntary movements of an infant according to another embodiment, comprises: a memory; and at least one processor connected to the memory, wherein the at least one processor may be configured to: receive a two-dimensional video of the infant moving voluntarily; generate the position coordinate data of joints of the infant for each frame of the video using a deep learning-based pose prediction model; and quantify the smoothness of the voluntary movements of the infant based on the position coordinate data of the joints.

Wherein the at least one processor may be further configured to generate angle data and angular velocity data for the joint based on the position coordinate data of the joints, after generating the position coordinate data.

Wherein the at least one processor may be configured to quantify the smoothness of the voluntary movements of the infant based on the angle data and the angular velocity data of the joints.

Wherein the at least one processor may be configured to measure a log dimensionless jerk (LDJ) and spectral arc length (SPARC) based on the angle data and the angular velocity data of the joints.

Wherein the position coordinate data for the joints may comprise, the position coordinate data for at least one of the left shoulder joint, the right shoulder joint, the left elbow joint, the right elbow joint, the left wrist joint, the right wrist joint, the left hip joint, the right hip joint, the left knee joint, the right knee joint, the left ankle joint, and the right ankle joint.

A computer-readable storage medium according to another embodiment may record a program for performing the method described in the above embodiments.

### ADVANTAGEOUS EFFECT

A method and system for automatically evaluating smoothness of voluntary movements of an infant of the present invention, enables the assessment of risk of cerebral palsy or developmental delay of an infant simply by recording a video of the infant moving voluntarily.

A method and system for automatically evaluating smoothness of voluntary movements of an infant of the present invention, has identified "smoothness" as a new parameter associated with the risk of cerebral palsy or developmental delay in infants. By quantifying the smoothness of voluntary movements of an infant through a machine learning-based automated approach, the risk of cerebral palsy or developmental delay in infants can be quantitatively assessed regardless of the evaluator's level of expertise.

According to the present invention, an effective method and system for automatically evaluating the smoothness of voluntary movements of an infant can be provided, enabling the early prediction of cerebral palsy or developmental delay in infants through a machine learning-based quantitative method.

The effects of the present invention are not limited to those described above, and effects not mentioned herein will be clearly understood by those skilled in the art to which the present invention pertains from the description and the accompanying figures.

### BRIEF DESCRIPTION OF THE DRAWINGS

FIG. 1 is a diagram illustrating the series of steps for obtaining angle data and angular velocity data of an infant of joints.
FIG. 2 is a diagram showing a measurement of spectral arc length based on the angle and the angular velocity data of shoulder and knee joints of infants in a normal group and a group with delayed motor development.
FIG. 3 is a diagram illustrating a method for automatically evaluating smoothness of voluntary movements of an infant.
FIG. 4 is a diagram illustrating a system for automatically evaluating smoothness of voluntary movements of an infant.

### BEST MODE FOR CARRYING OUT THE INVENTION

The following describes in detail a preferred embodiment of the present invention with reference to the accompanying figures. The advantages and features of the present invention, as well as the methods for achieving them, will become clear upon reference to the embodiments described in detail below, together with the accompanying figures. However, the present invention is not limited to the embodiments disclosed below but may be implemented in various different forms; these embodiments are provided merely to ensure a complete disclosure of the present invention and to fully inform a person skilled in the art of the scope of the invention, and the present invention is defined solely by the scope of the claims. Throughout the specification, the same reference numerals denote the same components.

Unless otherwise defined, all terms used in this specification (including technical and scientific terms) shall be used in the sense commonly understood by those skilled in the art to which the present invention pertains. Furthermore, terms defined in commonly used dictionaries shall not be interpreted broadly or excessively unless explicitly defined otherwise.

The terms used in this specification are intended to describe the embodiments and are not intended to limit the scope of the present invention. In the specification, the singular form includes the plural form unless otherwise specified. The terms "comprise" and/or "comprising" used in the specification do not preclude the presence or addition of one or more other components in addition to the components mentioned.

Unless otherwise stated in the specification, the terms "connected" or "linked" may include a situation where one element/feature is directly connected or linked to another element/feature, or indirectly connected or linked through an intervening element/feature, and do not necessarily refer solely to direct mechanical connection or linkage. Therefore, while the various schematic diagrams shown in the figures illustrate exemplary arrangements of elements and components, additional intervening elements, devices, features, or components may be present in actual embodiments (provided that the functionality of the shown elements is not adversely affected).

Furthermore, in the specification, the terms "transmitting" or "receiving" refer not only to the direct transmission or reception of information between a sender and a receiver, but may also include the transmission or reception of information via an intermediary, unless otherwise specified.

The embodiments of the present invention will now be described in detail with reference to the figures.

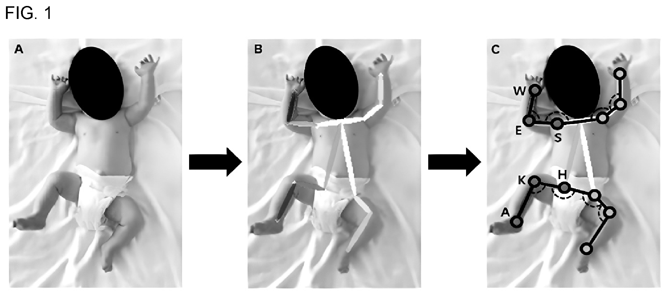

FIG. 1 is a diagram illustrating the series of steps for obtaining angle data and angular velocity data of an infant of joints.

First, A in FIG. 1 represents a two-dimensional video of an infant moving voluntarily. Two-dimensional videos of infants moving voluntarily can be recorded using a smartphone or camera. In the two-dimensional videos of infants moving voluntarily, it is desirable for the infant's head, arms, and legs to all be included within the video frame. In two-dimensional videos of infants moving voluntarily, it is desirable for the infant to be lying flat on their back, wearing minimal clothing, and fully awake. Furthermore, it is desirable for the infant to be in a comfortable, quiet, and undisturbed environment at a normal temperature, where the infant can move their arms, legs, and torso freely. The two-dimensional video of an infant moving voluntarily may be at least 3 minutes long, and preferably between 3 and 5 minutes in length.

Next, B in FIG. 1 represents the process of obtaining the position coordinate data of the infant's joints for each frame of the video using a deep learning-based pose prediction model. The present invention may use AlphaPose, a deep learning-based pose estimation algorithm, as the pose prediction model. AlphaPose is a pre-trained pose prediction model based on a Convolutional Neural Network (CNN) architecture. When a two-dimensional video of an infant moving voluntarily is input into AlphaPose, AlphaPose can estimate the infant's pose in real time and automatically obtain the position coordinate data of the infant's joints for each frame of the video.

Finally, C in FIG. 1 represents the process of obtaining angle data of the infant's joint and angular velocity data of the joint based on the position coordinate data of the infant's joint obtained for each frame of the video. The present invention may automatically obtain the angle data and angular velocity data of the joints of bilateral shoulders (S), elbows (E), wrist (W), hip (H) and knee (K) based on the joint position coordinate data of the bilateral shoulder (S), elbow (E), hip (H), , knee (K) and ankle (A) of the infant.

The present invention can automatically obtain the angles and angular velocity data of joints of the infant using functions related to the angle R.

The angle-related function of R in the present invention is a function provided by the R programming language and can perform angle-related measurements.

In the present invention, a joint angle may refer to the angle formed by the positional coordinates of two adjacent joints relative to a specific joint. For example, the angle of the left shoulder joint may refer to the angle formed by the right shoulder joint and the left elbow joint relative to the left shoulder joint. The angle of the right shoulder joint may refer to the angle formed by the left shoulder joint and the right elbow joint relative to the right shoulder joint. The angle of the left elbow joint may refer to the angle formed by the left shoulder joint and the left wrist joint relative to th the left elbow joint. The angle of the right elbow joint may refer to the angle formed by the right shoulder joint and the right wrist joint relative to the right elbow joint. The angle of the left hip joint may refer to the angle formed by the right hip joint and the left knee joint relative to the left hip joint. The angle of the right hip joint may refer to the angle formed by the left hip joint and the right knee joint relative to the right hip joint. The angle of the left knee joint may refer to the angle formed by the left hip joint and the left ankle joint relative to the left knee joint. The angle of the right knee joint may refer to the angle formed by the right hip joint and the right ankle joint relative to the right knee joint.

The angular velocity of a joint in the present invention can represent the rate of change of the joint angle over time. For example, the angular velocity of the left shoulder joint can represent the rate of change of the left shoulder joint over time. The angular velocity of the right shoulder joint can represent the rate of change of the right shoulder joint over time. The angular velocity of the left elbow joint can represent the rate of change of the left elbow joint over time. The angular velocity of the right elbow joint can represent the rate of change of the right elbow joint over time. The angular velocity of the left hip joint can represent the rate of change of the left hip joint over time. The angular velocity of the right hip joint can represent the rate of change of the right hip joint over time. The angular velocity of the left knee joint can represent the rate of change of the left knee joint over time. The angular velocity of the right knee joint can represent the rate of change of the right knee joint over time.

Previously, there have been few attempts to evaluate voluntary movements using joint angle data and joint angular velocity data rather than joint position coordinate data in infants. Since positional coordinates are highly influenced by factors such as the camera's height and angle relative to the object, whereas angles and angular velocities are relatively less affected, the present invention measured log dimensionless jerk (LDJ) and spectral arc length (SPARC) based on the infants' joint angle data and angular velocity data.

The LDJ of the present invention can be defined by Equations 1 and 2 below (see Balasubramanian *et al.,* 2012). First, Equation 1 is as follows. $DLJ ≜ - \frac{{\left({t}_{2}-{t}_{1}\right)}^{5}}{{{v}^{2}}_{peak}} {\int }_{{t}_{1}}^{{t}_{2}} {\left|\frac{{d}^{2} v \left(t\right)}{{dt}^{2}}\right|}^{2} dt$

The dimensionless jerk (DLJ) of the present invention can represent the rate of change of acceleration of a motion. Referring to Equation 1, the DLJ of the present invention can be defined as the negative of the value obtained by multiplying the integral of the square of the rate of change of acceleration by the cube of the duration of the movement, and then dividing this by the square of the maximum velocity of the joint angle.

Equation 2 is as follows. $LDJ ≜ - ln \left|DLJ\right|$

Referring to Equation 2, the LDJ of the present invention can be defined as the negative logarithm of the DLJ (Guide and Hermsdörfer, 2018). For example, in the present invention, Python (version 3.10; Python Software Foundation, Wilmington, DE, USA) can be used to measure the LDJ.

Since LDJ is more sensitive to small changes in movement than DLJ, it may be preferable to use LDJ rather than DLJ as an indicator for evaluating the voluntary movements of the infant (see Balasubramanian *et al.,* 2015; Hogan and Sternad, 2009).

The SPARC of the present invention can be defined by Equations 3 and 4 below (see: Balasubramanian *et al.,* 2012). Equation 3 is as follows. $SPARC ≜ - {\int }_{0}^{{ω}_{c}} {\left[{\left(\frac{1}{{ω}_{c}}\right)}^{2}+{\left(\frac{d \hat{V} \left(ω\right)}{dω}\right)}^{2}\right]}^{\frac{1}{2}} dω ; \hat{V} \left(ω\right) = \frac{V \left(ω\right)}{V \left(0\right)}$

In Equation 3, *V*(*ω*) represents the Fourier magnitude spectrum in the frequency domain of the motion, and *ω_{C}* can represent the cutoff frequency. *ω_{C}* can be defined by Equation 4 below. ${ω}_{c} ≜ min \left\{{{ω}_{c}}^{max}, min\left\{ω, \hat{V} \left(r\right)<\overline{V} ∀ r>ω\right\}\right\}$

In other words, the SPARC of the present invention is defined by *V*(*ω*) and *ω_{C}*, and the SPARC can refer to the arc length of the Fourier magnitude spectrum. For example, in the present invention, Python (version 3.10; Python Software Foundation, Wilmington, DE, USA) can be used to measure the SPARC.

In the following, a specific experimental example is described in which the smoothness of voluntary movements of an infant is quantied based on LDJ and SPARC measured for angle data and angular velocity data of joint of the infant, and the risk of cerebral palsy or motor development delay in the infant is evaluated.

In the present invention, a total of 111 infants were recruited, including preterm infants born before 32 weeks of gestation and low-birth-weight infants weighing less than 1,500 grams. However, infants with genetic syndromes or major congenital malformations were excluded.

In the present invention, first, a two-dimensional video of an infant moving voluntarily was recorded for an infant who had reached a specific age. However, to more accurately evaluate the risk of cerebral palsy or motor development delay, the term-equivalent age was considered based on the due date rather than the actual age at birth for premature infants. Subsequently, LDJ and SPARC were measured for the infants' joint angle data and joint angular velocity data; Table 1 below presents the results.

**Table 1**

| | | **Normal (n=100)** | **Delayed Motor Development (n=11)** | **p-value** |
|---|---|---|---|---|
| **LDJ** | **right shoulder** | **-22.97 (0.84)** | **-22.02 (1.33)** | **0.007*** |
| | **left shoulder** | **-22.89 (0.84)** | **-22.07 (1.38)** | **0.059** |
| | **right elbow** | **-23.04 (0.91)** | **-22.02 (1.45)** | **0.016*** |
| | **left elbow** | **-23.05 (0.82)** | **-22.04 (1.43)** | **0.017*** |
| | **right hip** | **-22.99 (0.82)** | **-22.15 (1.28)** | **0.035*** |
| | **left hip** | **-22.96 (0.86)** | **-22.32 (1.14)** | **0.106** |
| | **right knee** | **-23.02 (0.81)** | **-22.08 (1.39)** | **0.023*** |
| | **left knee** | **-23.03 (0.81)** | **-22.26 (1.36)** | **0.053** |
| **SPARC** | **right shoulder** | **-33.91 (8.85)** | **-27.09 (10.92)** | **0.041*** |
| | **left shoulder** | **-33.44 (8.64)** | **-29.07 (11.19)** | **0.125** |
| | **right elbow** | **-34.26 (9.30)** | **-28.64 (10.70)** | **0.093** |
| | **left elbow** | **-33.11 (8.79)** | **-27.89 (15.95)** | **0.161** |
| | **right hip** | **-33.4 (8.71)** | **-27.63 (11.18)** | **0.043*** |
| | **left hip** | **-33.66 (9.41)** | **-29.70 (9.44)** | **0.188** |
| | **right knee** | **-32.86 (9.09)** | **-26.89 (10.89)** | **0.045*** |
| | **left knee** | **-33.84 (9.61)** | **-25.25 (9.57)** | **0.006*** |

Table 1, shows the mean values (standard deviation) of LDJ and SPARC measured at the right shoulder, left shoulder, right elbow, left elbow, right hip, left hip, right knee, and left knee joints for 100 infants in the normal group without motor development delays and 11 infants in the group with motor development delays. The p-value is a concept used in statistical hypothesis testing; if the p-value is less than the significance level (e.g., 0.05), it means that the observed results are unlikely to have occurred by chance.

Referring to Table 1 above, the LDJ values for the right shoulder, right elbow, left elbow, right hip, and right knee joints of infants in the group with motor development delay were measured at -22.97, 23.04, 23.05, -22.99, -23.02, respectively, while the LDJ values for the right shoulder, right elbow, left elbow, right hip, and right knee joints of infants in the normal group were -22.02, -22.02, -22.04, - 22.15, and -22.08, respectively. In other words, the LDJ values of infants in the group with motor development delay was significantly higher than that of infants in the normal group at most joints. Thus, it was found that the LDJ values measured at the infants' joints showed a significant positive correlation with the likelihood of subsequent motor development delay in the infants

In addition, the SPARC values for the right shoulder, right hip, right knee, and left knee joints of infants in the group with motor development delays were measured at -33.91, -33.46, -32.86, and -33.84, respectively, while the SPARC values for the infants in the normal group at the right shoulder, right hip, right knee, and left knee joints were -27.09, -27.63, -26.89, and -25.25, respectively. In other words, the SPARC values for infants in the group with motor development delay were significantly higher than those of infants in the normal group at the right shoulder, right hip, right knee, and left knee joints. Thus, it was found that SPARC values at the infant's joints also showed a significant positive correlation with the likelihood of subsequent motor development delay in the infant.

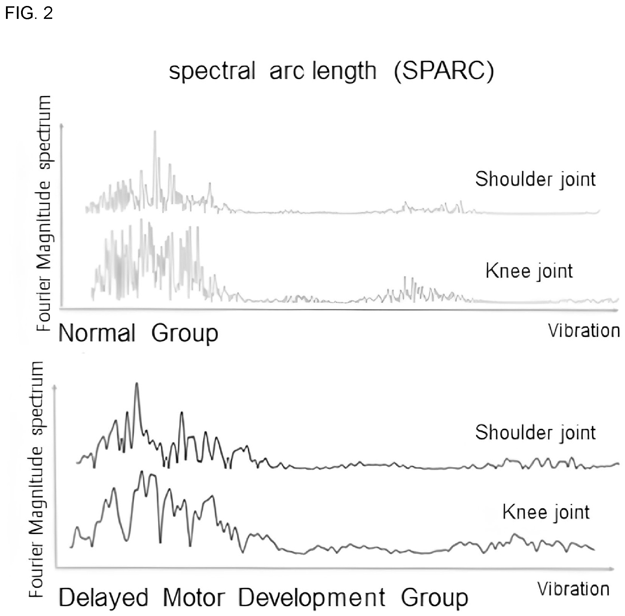

These characteristics can also be seen in FIG. 2. FIG. 2 shows the SPARC values for the shoulder and knee joints of infants in the normal group and those in the group with delayed motor development. As shown in FIG. 2, the smoothness of movement in the shoulder and knee joints of infants in the group with delayed motor development was found to be greater than that of infants in the normal group. In other words, it was found that the smoothness of movement (LDJ and SPARC) in the joints of infants in the group with motor development delay was greater than that in the joints of infants in the normal group.

Furthermore, in the present invention, a prospective longitudinal cohort study was conducted on 111 infants to determine whether the smoothness of voluntary movements of an infant serves as an accurate indicator of the risk of subsequent motor developmental delay. Specifically, when the 111 infants reached 9 months of adjusted age, their motor composite scores were measured using the motor development test kit defined in the Bayley Scales of Infant and Toddler Development, Third Edition (BSID-III). In the present invention, it was confirmed that the motor composite scores based on the Bayley Scales of Infant and Toddler Development, Third Edition, for infants in the group with motor developmental delay were 80 points or lower when rehabilitation support was provided.

Table 2 below shows the correlation between LDJ and SPARC values measured at the joints of the infant and the motor composite scores of the infant based on the Bayley Scales of Infant and Toddler Development, Third Edition (BSID-III). The correlation coefficients in Table 2, expressed as Pearson correlation coefficients or Spearman correlation coefficients, were obtained by analyzing the correlation between the absolute values of LDJ and SPARC measured in the joint of the infant and the motor composite scores of the infant based on the Bayley Scales of Infant and Toddler Development, Third Edition (BSID-III). This statistical analysis was performed using SPSS software (version 26; SPSS Inc., Chicago, IL, USA). The p-value is a concept used in statistical hypothesis testing; if the p-value is less than the significance level (e.g., 0.05), it means that the observed results are unlikely to have occurred by chance.

**Table 2**

| | **correlation coefficient** | | **p-value** |
|---|---|---|---|
| **LDJ** | **right shoulder** | **0.208** | **0.029*** |
| | **left shoulder** | **0.117** | **0.223** |
| | **right elbow** | **0.088** | **0.368** |
| | **left elbow** | **0.16** | **0.093** |
| | **right hip** | **0.139** | **0.147** |
| | **left hip** | **0.053** | **0.582** |
| | **right knee** | **0.167** | **0.079** |
| | **left knee** | **0.076** | **0.428** |
| **SPARC** | **right shoulder** | **0.318** | **0.001*** |
| | **left shoulder** | **0.119** | **0.212** |
| | **right elbow** | **0.111** | **0.246** |
| | **left elbow** | **0.169** | **0.076** |
| | **right hip** | **0.211** | **0.026*** |
| | **left hip** | **0.118** | **0.219** |
| | **right knee** | **0.234** | **0.014*** |
| | **left knee** | **0.219** | **0.021*** |

Referring to Table 2 above, the absolute value of LDJ at the infant's right shoulder joint and the infant's motor composite score based on the Bayley Scales of Infant and Toddler Development, Third Edition (BSID-III), showed a correlation coefficient of 0.208. Additionally, the absolute values of LDJ at the infant's right shoulder, right hip, right knee, and left knee joints, and the infant's motor composite score based on the Bayley Scales of Infant and Toddler Development, Third Edition (BSID-III), showed correlation coefficients of 0.318, 0.211, 0.234, and 0.219, respectively. This indicates that as the absolute values of movement smoothness (LDJ and SPARC) at the infant's joints increase, the infant's motor composite score based on the Bayley Scales of Infant and Toddler Development, Third Edition (BSID-III), also increases. In other words, the absolute values of the smoothness of voluntary movements (LDJ and SPARC) of the infant are positively correlated with the motor composite scores based on the the Bayley Scales of Infant and Toddler Development, Third Edition (BSID-III), for infants at 9 months of adjusted age. Thus, in the present invention, it was discovered that the smoothness of voluntary movements of the infant is an important parameter associated with the risk of subsequent motor developmental delay in infants.

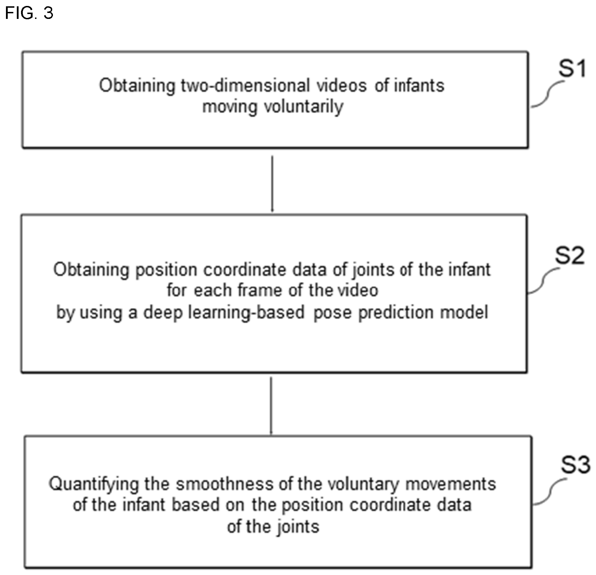

FIG. 3 is a diagram illustrating a method for automatically evaluating smoothness of voluntary movements of an infant.

Referring to FIG. 3, the method for automatically evaluating the smoothness of voluntary movements of an infant may comprises the steps of: obtaining a two-dimensional video of the infant who is moving voluntarily (S1); obtaining position coordinate data of joints of the infant for each frame of the video by using a deep learning-based pose prediction model (S2); and quantifying the smoothness of the voluntary movements of the infant based on the position coordinate data of the joints (S3).

In Step S1, two-dimensional videos of infants who is moving voluntarily can be obtained. In Step S2, using a deep learning-based pose prediction model, position coordinate data of joints of the infant can be obtained for each frame of the two-dimensional video of the infant moving voluntarily. In Step S3, the smoothness of voluntary movements of the infant can be quantified based on the position coordinate data of the joints.

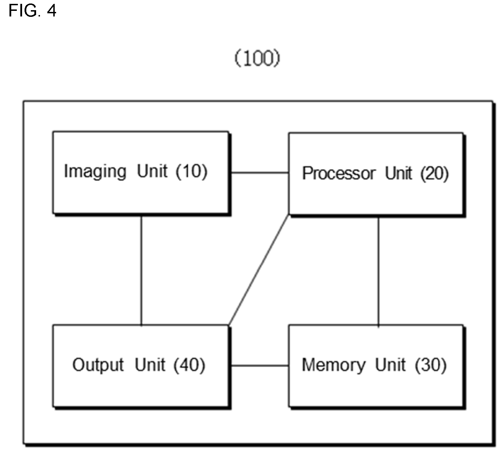

FIG. 4 is a diagram illustrating a system for automatically evaluating smoothness of voluntary movements of an infant.

Referring to FIG. 4, the system (100) for automatically evaluating smoothness of voluntary movements of an infant may comprise an imaging unit (10), a processor unit (20), a memory unit (30), and an output unit (40).

The imaging unit (10) is capable of capturing the voluntary movements of an infant lying flat on their back. The imaging unit (10) may comprise a camera mounted on a tablet, smartphone, or similar device, etc.

The processor unit (20) may correspond to processors (20) found in various types of computing devices, such as personal computers (PCs), server devices, mobile devices, embedded devices, and Internet of Things (IoT) devices. For example, the processor unit (20) may correspond to a central processing unit (CPU) graphics processing unit (GPU), application processor (AP), neural processing unit (NPU), or similar processors (20), but is not limited thereto.

The processor unit (20) may be implemented as an array of multiple logic gates, or as a combination of a general-purpose microprocessor and the memory unit (30) in which programs executable by the microprocessor are stored. Furthermore, those skilled in the art will understand that it may also be implemented using other forms of hardware.

The memory unit (30) may be electrically connected to the processor unit (20). The memory unit (30) is hardware that stores various types of data processed by the processor unit (20); for example, it can store two-dimensional video data of an infant moving voluntarily.

The memory unit (30) may comprise at least one of volatile memory or nonvolatile memory. The nonvolatile memory comprises Read-Only Memory (ROM), Programmable Read-Only Memory (PROM), Electrically Programmable Read-Only Memory (EPROM), Electrically Erasable and Programmable Read-Only Memory (EEPROM), flash memory, Phase-Change RAM (PRAM), Magnetic RAM (MRAM), Resistive RAM (RRAM), Ferroelectric RAM (FeRAM), and the like. The volatile memory comprises Dynamic RAM (DRAM), Static RAM (SRAM), Synchronous DRAM (SDRAM), PRAM, Magnetic RAM (MRAM), Resistive RAM (RRAM), and the like. In the embodiment, the memory unit (30) may be implemented as at least one of a Hard Disk Drive (HDD), Solid State Drive (SSD), Compact Flash (CF), Secure Digital (SD), Micro Secure Digital (Micro-SD), Mini Secure Digital (Mini-SD), Extreme Digital (xD), or Memory Stick.

The output unit (40) may be electrically connected to the imaging unit (10), the processor unit (20), and the memory unit (30). The output unit (40) may be a visual interface. Thus, the method and system for automatically evaluating the smoothness of voluntary movements of an infant according to the present invention can provide the benefit of assessing the risk of cerebral palsy or developmental delay in an infant simply by capturing video of the infant moving voluntarily.

In particular, the method and system for automatically evaluating the smoothness of voluntary movements of an infant according to the present invention, can provide the benefit of quantitatively evaluating the risk of cerebral palsy or developmental delay in infants, regardless of the evaluator's level of expertise, by quantifying the smoothness of voluntary movements of the infant through a machine learning-based automated approach.

In other words, the method and system for automatically evaluating the smoothness of voluntary movements of an infant according to the present invention, can provide the benefit of enabling the early prediction of cerebral palsy or developmental delay in infants through a machine learning-based quantitative method.

Meanwhile, the order of the operations described in the methods, systems, and processes disclosed in this specification is provided by way of example. Therefore, the order of the steps may be adjusted as necessary within the scope of the invention. Furthermore, the devices and systems disclosed in this specification may comprise means capable of performing the functions described herein, and may be implemented as standalone devices or systems, or may exist in a form that is interconnected with or integrated into other systems, as needed.

The operations described with reference to FIGs 1 through 4 may be performed or implemented on a computer-readable storage medium on which the program is recorded. Furthermore, the instructions stored on the computer-readable medium may cause a processor in each device or system to perform the methods and processing associated with those instructions.

By way of non-limiting examples, computer-readable media may be used to store in the form of RAM, ROM, EEPROM, CD-ROM or other optical disk storage, magnetic disk storage or other magnetic storage devices, or desired program code instructions or data structures, and may comprise any other storage medium accessible by a general-purpose or special-purpose computer, or a general-purpose or special-purpose processor. The terms "disk" and "disc," as used herein, comprise compact discs (CDs), laser discs, optical discs, digital versatile discs (DVDs), floppy disks, and Blu-ray discs; the disks typically reproduce data magnetically, whereas the discs reproduce data optically using a laser. Combinations of the foregoing should also be comprised within the scope of computer-readable storage media.

Although the embodiments of the present invention have been described with reference to the accompanying figures, those skilled in the art will understand that the present invention may be practiced in other specific forms without departing from its technical concept or essential features. Therefore, the embodiments described above should be understood as illustrative in all respects and not as limiting.

## Claims

1. A method for automatically evaluating smoothness of voluntary movements of an infant, comprises the steps of:
obtaining a two-dimensional video of the infant who is moving voluntarily;
obtaining position coordinate data of joints of the infant for each frame of the video by using a deep learning-based pose prediction model; and
quantifying the smoothness of the voluntary movements of the infant based on the position coordinate data of the joints,
as a method for automatically evaluating the smoothness of voluntary movements of an infant performed by a processor.

2. The method for automatically evaluating smoothness of voluntary movements of an infant according to claim 1, further comprises the steps of:
obtaining angle data and angular velocity data of the joints based on the position coordinate data of the joints, after obtaining the position coordinate data of the joints,

3. The method for automatically evaluating smoothness of voluntary movements of an infant according to claim 2,
wherein the quantifying the smoothness of the voluntary movements of the infant comprises,
quantifying the smoothness of the voluntary movements of the infant based on the angle data and the angular velocity data of the joints.

4. The method for automatically evaluating smoothness of voluntary movements of an infant according to claim 3,
wherein the quantifying the smoothness of the voluntary movements of the infant comprises,
measuring a log dimensionless jerk (LDJ) and spectral arc length (SPARC) based on the angle data and the angular velocity data of the joints.

5. The method for automatically evaluating smoothness of voluntary movements of an infant according to claim 1,
wherein the position coordinate data for the joints comprises, the position coordinate data for at least one of the left shoulder joint, the right shoulder joint, the left elbow joint, the right elbow joint, the left wrist joint, the right wrist joint, the left hip joint, the right hip joint, the left knee joint, the right knee joint, the left ankle joint, and the right ankle joint.

6. A system for automatically evaluating smoothness of voluntary movements of an infant comprises:
a memory; and
at least one processor connected to the memory,
wherein the at least one processor is configured to:
receive a two-dimensional video of the infant moving voluntarily;
generate the position coordinate data of joints of the infant for each frame of the video using a deep learning-based pose prediction model; and
quantify the smoothness of the voluntary movements of the infant based on the position coordinate data of the joints,
as a system for automatically evaluating smoothness of voluntary movements of an infant

7. The system for automatically evaluating smoothness of voluntary movements of an infant according to claim 6,
wherein the at least one processor is further configured to,
generate angle data and angular velocity data for the joint based on the position coordinate data of the joints.

8. The system for automatically evaluating smoothness of voluntary movements of an infant according to claim 7,
wherein the at least one processor is configured to,
quantify the smoothness of the voluntary movements of the infant based on the angle data and the angular velocity data of the joints.

9. The system for automatically evaluating smoothness of voluntary movements of an infant according to claim 8,
wherein the at least one processor is configured to,
measure a log dimensionless jerk (LDJ) and spectral arc length (SPARC) based on the angle data and the angular velocity data of the joints.

10. The system for automatically evaluating smoothness of voluntary movements of an infant according to claim 6,
wherein the position coordinate data for the joints comprises, the position coordinate data for at least one of the left shoulder joint, the right shoulder joint, the left elbow joint, the right elbow joint, the left wrist joint, the right wrist joint, the left hip joint, the right hip joint, the left knee joint, the right knee joint, the left ankle joint, and the right ankle joint.

11. A computer-readable storage medium on which is recorded a program that performs the method described in any one of claims 1 to 5.
